# EUROPEAN PATENT SPECIFICATION

(11) **EP 1 587 455 B1**
(45) Date of publication and mention of the grant of the patent: **14.03.2007**
(21) Application number: 03703587.0
(22) Date of filing: 31.01.2003
(51) Int. Cl.: A61F 2/26, A61F 5/41

(54) **ELECTRICALLY OPERABLE IMPOTENCE TREATMENT APPARATUS**
ELEKTRISCH BEDIENBARE VORRICHTUNG ZUR IMPOTENZBEHANDLUNG
APPAREIL DE TRAITEMENT DE L'IMPOTENCE A FONCTIONNEMENT ELECTRIQUE

(43) Date of publication of application: 26.10.2005
(73) Proprietor: Potencia Medical AG, 6341 Baar (CH)
(72) Inventor: FORSELL, Peter, CH-6300 Zug (CH)
(74) Representative: Strandin, Heléne
(86) International application number: PCT/SE2003/000169
(87) International publication number: WO 2004/066879

(56) References cited:
- WO-A1-00/15158
- WO-A1-01/12078
- WO-A2-01/47434
- FR-A1- 2 688 693

## Description

The present invention relates to a male sexual impotence treatment apparatus, comprising a constriction device implantable in a male patient, who suffers from sexual impotence, for engaging the patient's penile tissue or the prolongation thereof, and an implantable electrically powered operation device that operates the constriction device to temporarily constrict the penile tissue or the prolongation thereof to restrict the penile exit blood flow to achieve erection. The apparatus further comprises an electric power supply adapted to be implanted in the patient remote from the operation device, and an insulated electric wire connecting the electric power supply and the operation device. Such a device is described in WO-A- 01/47434.

The expression "penile tissue or the prolongation thereof" should be understood to mean the penile tissue extended inside the human body and following the pathway of the blood flow leaving the penis i.e. one or more exit veins from the penis, the corpus cavernosum, crura or the prolongation thereof.

Male sexual impotence is a widespread problem. Many different solutions to this problem have been tried. A main solution currently practised and disclosed in for instance U.S. Patent Nos. 5437605 and 4841461 is to implant a hydraulic inflatable silicone prosthesis in the cavities of the corpora cavernosa of the patient's penis. In fluid connection with this prosthesis is a reservoir implanted in the scrotum. By manual pumping action the prosthesis is filled with fluid from the reservoir to effect erect penile condition or is emptied of fluid, which returns to the reservoir, to effect flaccid penile condition. However, there are several more or less severe disadvantages of this main solution. Above all, the penis is more or less damaged by the operation and it is practically impossible to reverse the operation. Another disadvantage is that rather strong forces act against this implanted prosthesis resulting in a significant risk of the prosthesis being broken.

Another solution to achieve erection is to restrict the blood flow leaving the penis. For example, U.S. Patent No. 4829990 discloses two hydraulically operated inflatable cuffs wrapped around the respective crura. A disadvantage of such a solution is that it involves complicated surgery. Another example on this solution is given by U.S. Patent No. 4828544, which discloses an artificial fistula system surgically implanted and providing a primary fistula between the femoral artery and the femoral vein and a secondary fistula for leading blood from the primary fistula to the penis. An inflatable balloon engages the primary fistula between the secondary fistula and the vein. The balloon is in fluid connection with a manually compressible reservoir implanted in the scrotum. Again, implantation of this artifical fistula system requires delicate surgery.

Yet another example on the blood flow restriction solution is given by WO 01/54626, which discloses an elongate hydraulically adjustable constriction member adapted to temporarily constrict the penile tissue or the prolongation thereof of a patient's penile tissue to restrict the blood flow leaving the penis to achieve erection. There are an adjustment device that includes an inflatable cavity in the elongate constriction member and hydraulic means for adding hydraulic fluid to and withdrawing hydraulic fluid from the inflatable cavity.

A mechanical apparatus for constricting the penile tissue or the prolongation thereof may typically involve an electrically powered operation device connected to the constriction device. Therefore, such an operation device would be located at the patient's penile tissue. On the other hand, the electric power supply, for example a battery, necessary for powering the operation device with electric energy should be implanted subcutaneously at a suitable location, which would be remote from the operation device, in order to permit easy access from outside the patient's body for service or replacement of the power supply. In consequence, it would be necessary to implant a fairly long electric wire for connecting the power supply to the operation device. However, considering the fact that the apparatus is to be implanted for many years, possibly for the rest of the patient's life, the use of such an electric wire would make the apparatus unreliable, because the movements of the penile tissue or the prolongation thereof might sooner or later break the electric wire.

The object of the present invention is to provide a new reliable electrically operable apparatus for constricting the penile tissue or the prolongation thereof of an impotent patient.

According to claim 1, the inventive object is obtained by an apparatus of the kind presented initially characterised in that the electric wire is resilient and extends helically between the electric power supply and the electrically powered operation device. As a result, the resilient helically extending electric wire is capable of addressing the movements from the penile tissue or the prolongation thereof, because it may easily be temporarily extended without risking breakage.

The constriction device may be implanted in the base of the patient's penis or the prolongation thereof and preferably may engage the corpus cavernosum, crura or the prolongation thereof of the penis. However, there are several alternative positions of the constriction device that give more or less satisfactory restriction of the blood flow leaving the penis. Thus, as a first alternative the constriction device may extend around both corpora cavernosa or crura of the penis as a single unit. As a second alternative the constriction device may comprise two elongated constriction members extending around the respective corpora cavernosa or crura. As a third alternative an elongated constriction member of the constriction device may encircle one or more of the penile exit veins. As a fourth alternative the constriction device may comprise several constriction members extending around the respective penile exit veins.

The electric wire should be designed to conduct a current in the order of milliamperes, which is needed for powering the operation device when operating the constriction device, so that the constriction device exerts a force strong enough to constrict the penile tissue or the prolongation thereof, so that the patient's penile exit blood flow is restricted. Thus, the electric wire should have a low resistance.

Advantageously, the apparatus may include an energy transforming device capable of transforming wireless energy transmitted from outside the patient's body into electric energy, whereby the need for regularly replacing the electric power supply, when it is depleted, is eliminated.

Preferably, the operation device includes an electric motor connected to the electric power supply by means of the helical electrical wire. The constriction device may include a hydraulic constriction device inflatable by hydraulic fluid, and the operation device may include an electrically driven pump (for example driven by the electric motor) hydraulically connected to the hydraulic constriction device.

In accordance with a preferred embodiment of the invention, the constriction device is non-inflatable. In this embodiment, the constriction device comprises an elongate constriction member adapted to extend around the penile tissue or the prolongation thereof. The constriction member includes a main portion and two elongated end portions, and the operation device establishes longitudinal relative displacement between the end portions of the constriction member to constrict or release the penile tissue or the prolongation thereof. The operation device may include a movement transferring means in engagement with at least one of the end portions of the constriction member and operable by the electric motor to displace the one end portion relative to the other end portion of the constriction member. The movement transferring means may include a gear wheel fixed to the other end portion of the constriction member and a gear rack formed on the one end portion of the constriction member, the gear wheel and the gear rack being in mesh with each other. The operation device may further include a worm gear connected between the electric motor and the gear wheel.

Generally, the constriction device includes a housing containing the operation device, and the elongate constriction member and the housing are adapted to form a loop around the patient's penile tissue or the prolongation thereof.

Advantageously, the apparatus includes a control device, suitably in the form of a wireless remote control, for controlling the operation device to operate the constriction device. The control device may include an internal, preferably programmable, control unit implantable in the patient for controlling the operation device. Furthermore, the control device may include an external control unit outside the patient's body, wherein the internal control unit is programmable by the external control unit.

The apparatus may include at least one implantable sensor for sensing at least one physical parameter of the patient. The sensor may be a pressure sensor for directly or indirectly sensing the pressure against the constriction device. The control device may suitably control the operation device in response to signals from the sensor. Where the control device includes an internal control unit, the internal control unit may control the operation device in response to signals from the sensor.

The apparatus may include a switch implantable in the patient for directly or indirectly switching the electric energy supplied by the electric power supply.

### In the enclosed drawings:

Figure 1 is a schematic view of an apparatus according to the present invention implanted in a male impotent patient, and
Figure 2 schematically illustrates an embodiment of a constriction device of the apparatus for use in the embodiment of Figure 1.

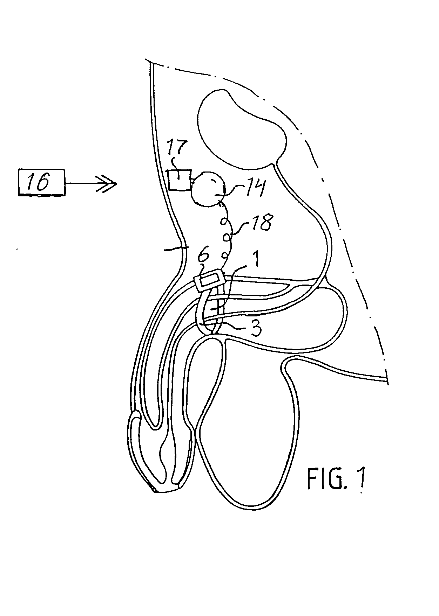

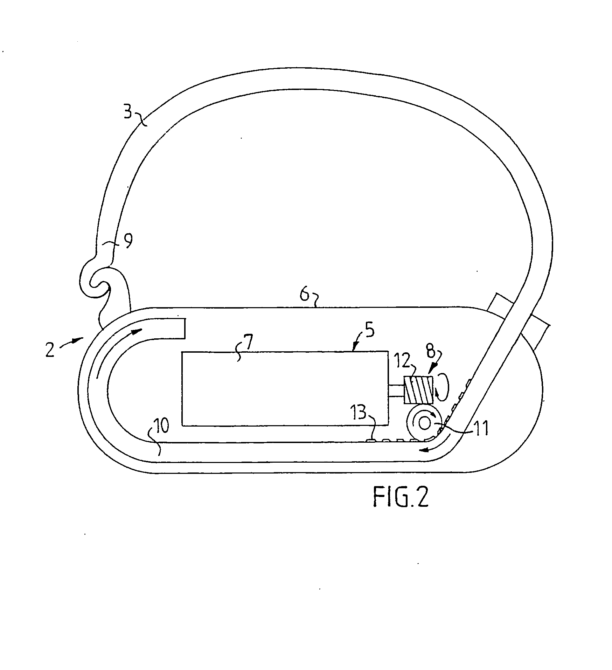

Figure 1 shows an apparatus of the present invention for treating an impotent patient comprising a constriction device 2 applied on the patient's penile tissue 1. The constriction device 2 includes an elongate operable constriction member 3 extending in a loop around the penile tissue 1. An electrically powered operation device 5, which operates the constriction member 3 to temporarily constrict the penile tissue when erection is desired, is housed in an elongate housing 6, see Figure 2. The constriction member 3 has a first end portion 9 releasably connected to the housing 6 and a second end portion 10 connected to the operation device 5. The operation device 5 includes an electric motor 7 and a movement transferring means 8 in engagement with the end portion 10. The electric motor 7 operates the movement transferring means 8 to displace the end portion 10 relative to portion 9 in the loop formed by the constriction member 3. The movement transferring means 8 includes a gear wheel 11 fixed to the housing 6, a worm gear 12 connected between the electric motor 7 and the gear wheel 11, and a gear rack 13 formed on the end portion 10, wherein the gear wheel 11 and the gear rack 13 are in mesh with each other.

A rechargeable electric power supply 14 is subcutaneously implanted in the patient. An external remote control 16 controls the operation device 5 and transmits signals that are received by a combined control and energy transforming unit 17 subcutaneously implanted in the patient. The unit 17 is electrically connected to the electric power supply 14 and transforms the energy of the signals into an electric current that is used for charging the electric power supply 14. For example, the signals may include electromagnetic waves and the unit 17 may include an electric p-n junction element that transforms the wireless energy into an electric current.

A resilient insulated electric wire 18 connects the power supply 14 and the electric motor 7. The electric wire 18 extends helically between the power supply 14 and housing 6 that contains the motor 7, in order to permit the electric wire 18 to be temporarily extended when movements of the penile tissue occur, so that the risk of breaking the electric wire 18 is eliminated.

## Claims

1. A male sexual impotence treatment apparatus, comprising a constriction device (2) implantable in a male patient, who suffers from sexual impotence, for engaging the patient's penile tissue or the prolongation thereof, an implantable electrically powered operation device (5) that operates the constriction device to temporarily constrict the penile tissue or the prolongation thereof to restrict the penile exit blood flow to achieve erection, an electric power supply (14) adapted to be implanted in the patient remote from the operation device, and an insulated electric wire (18) connecting the electric power supply and the operation device, **characterized in that** the electric wire (18) is resilient and extends helically between the electric power supply (14) and the electrically powered operation device (5).

2. An apparatus according to claim 1, further comprising an implantable energy transforming device (17) capable of transforming wireless energy transmitted from outside the patient's body into electric energy.

3. An apparatus according to claim 1 or 2, wherein the operation device (5) comprises an electric motor (7) connected to the electric power supply (14) by means of the helical electrical wire (18).

4. An apparatus according to claim 3, wherein the constriction device (2) is non-inflatable.

5. An apparatus according to claim 4, wherein the constriction device (2) comprises an elongate constriction member (3) adapted to extend around the penile tissue or the prolongation thereof.

6. An apparatus according to claim 5, wherein the constriction member (3) comprises a main portion and two elongated end portions (9, 10), and the operation device (5) establishes longitudinal relative displacement between the end portions of the constriction member.

7. An apparatus according to claim 6, wherein the operation device (5) comprises an electric motor and a movement transferring means (8) in engagement with at least one (10) of the end portions of the constriction member (3) and operable by the electric motor (7) to displace the one end portion (10) relative to the other end portion (9) of the constriction member.

8. An apparatus according to claim 7, wherein the movement transferring means (8) comprises a gear wheel (11) fixed relative to the other end portion (9) of the constriction member (3) and a gear rack (13) formed on the one end portion (10) of the constriction member, the gear wheel and the gear rack being in mesh with each other.

9. An apparatus according to claim 8, wherein the operation device (5) comprises a worm gear (12) connected between the electric motor (7) and the gear wheel (11).

10. An apparatus according to any one of claims 1-3, wherein the constriction device (2) comprises a hydraulic constriction device and the operation device comprises an electrically driven pump hydraulically connected to the hydraulic constriction device.

11. An apparatus according to any one of claims 5-9, wherein the constriction device (2) comprises a housing (6) containing the operation device (5), and the elongate constriction member (3) and the housing are adapted to form a loop around the patient's penile tissue or the prolongation thereof.

12. An apparatus according to any one of claims 1-11, further comprising a wireless remote control (16) for controlling the operation device (5) to operate the constriction device (2).

13. An apparatus according to any one of claims 1-12 further comprising at least one implantable sensor for sensing at least one physical parameter of the patient.

14. An apparatus according to claim 13, wherein the sensor comprises a pressure sensor for directly or indirectly sensing the pressure against the constriction device (2).

15. An apparatus according to claim 14, further comprising a control device for controlling the operation device in response to signals from the sensor.

16. An apparatus according to claim 15, wherein the control device comprises an internal control unit implantable in the patient for controlling the operation device in response to signals from the sensor.

17. An apparatus according to any one of claims 1-14, further comprising a control device for controlling the operation device.

18. An apparatus according to claim 17, wherein the control device comprises an internal control unit implantable in the patient for controlling the operation device.

19. An apparatus according to claim 18, wherein the internal control unit is programmable.

20. An apparatus according to claim 19, wherein the control device comprises an external control unit outside the patient's body, the internal control unit being programmable by the external control unit.

21. An apparatus according to any one of claims 1-20, further comprising a switch implantable in the patient for directly or indirectly switching the electric energy supplied by the electric power supply.

## Patentansprüche

1. Vorrichtung zur Behandlung männlicher sexueller Impotenz, mit einer in einen unter sexueller Impotenz leidenden Patienten implantierbaren Konstriktionsvorrichtung (2) zum Angreifen an dem Penisgewebe des Patienten oder dessen Verlängerung, einer implantierbaren elektrischen Betätigungsvorrichtung (5), welche die Konstriktionsvorrichtung zum vorübergehenden Einschnüren des Penisgewebes oder dessen Verlängerung betätigt, um zum Zwecke einer Erektion den Blutfluss aus dem Penis zu begrenzen, einer elektrischen Energiequelle (14), die von der Betätigungsvorrichtung entfernt in den Patienten implantierbar ist, und einem isolierten elektrischen Draht (18), welcher die elektrische Energiequelle und die Betätigungsvorrichtung miteinander verbindet, **dadurch gekennzeichnet, dass** der elektrische Draht (18) elastisch ist und sich schraubenlinienförmig zwischen der elektrischen Energiequelle (14) und der elektrisch betriebenen Betätigungsvorrichtung (5) erstreckt.

2. Vorrichtung nach Anspruch 1, ferner mit einer implantierbaren Energieumwandelvorrichtung (17), welche derart ausgebildet ist, dass sie von außerhalb des Patientenkörpers zugeführte drahtlose Energie in elektrische Energie umwandelt.

3. Vorrichtung nach Anspruch 1 oder 2, bei der die Betätigungsvorrichtung (5) einen Elektromotor (7) aufweist, der mit der elektrischen Energiequelle (14) durch den schraubenlinienförmigen elektrischen Draht (18) verbunden ist.

4. Vorrichtung nach Anspruch 3, bei der die Konstriktionsvorrichtung (2) nicht aufblasbar ist.

5. Vorrichtung nach Anspruch 4, bei der die Konstriktionsvorrichtung (2) ein langgestrecktes Konstriktionselement (3) aufweist, das sich um das Penisgewebe oder dessen Verlängerung herum erstreckt.

6. Vorrichtung nach Anspruch 5, bei der das Konstriktionselement (3) einen Hauptbereich und zwei langgestreckte Endbereiche (9, 10) aufweist, und die Betätigungsvorrichtung (5) eine relative Längsverschiebung zwischen den Endbereichen des Konstriktionselements bewirkt.

7. Vorrichtung nach Anspruch 6, bei der die Betätigungsvorrichtung (5) einen Elektromotor und eine Bewegungsübertragungseinrichtung (8) aufweist, welche in Eingriff mit mindestens einem (10) der Endbereiche des Konstriktionselements (3) steht und von dem Elektromotor (7) zum Verschieben des einen Endbereichs (10 ) in bezug auf den anderen Endbereich (9) des Konstriktionselements betätigbar ist.

8. Vorrichtung nach Anspruch 7, bei der die Bewegungsübertragungseinrichtung (8) ein in bezug auf den anderen Endbereich (9) des Konstriktionselements (3) angebrachtes Zahnrad (11) und eine an dem einen Endbereich (10) des Konstriktionselements ausgebildete Zahnstange (13) aufweist, wobei das Zahnrad und die Zahnstange miteinander kämmen.

9. Vorrichtung nach Anspruch 8, bei der die Betätigungsvorrichtung (5) ein Schneckenrad (12) aufweist, das zwischen dem Elektromotor (7) und dem Zahnrad (11) angeordnet ist.

10. Vorrichtung nach einem der Ansprüche 1 - 3, bei der die Konstriktionsvorrichtung (2) eine hydraulische Konstriktionsvorrichtung und die Betätigungseinrichtung eine elektrisch angetriebene Pumpe aufweist, die hydraulisch mit der hydraulischen Konstriktionsvorrichtung verbunden ist.

11. Vorrichtung nach einem der Ansprüche 5-9, bei der die Konstriktionsvorrichtung (2) ein Gehäuse (6) aufweist, welches die Betätigungsvorrichtung (5) enthält, und das langgestreckte Konstriktionselement (3) und das Gehäuse zur Bildung einer Schlaufe um das Penisgewebe eines Patienten oder dessen Verlängerung ausgebildet sind.

12. Vorrichtung nach einem der Ansprüche 1-11, ferner mit einer drahtlosen Fernsteuerung (16) zum Steuern der Betätigungsvorrichtung (5) zum Betreiben der Konstriktionsvorrichtung (2).

13. Vorrichtung nach einem der Ansprüche 1-12, ferner mit mindestens einem implantierbaren Sensor zum Erkennen mindestens eines physischen Parameters des Patienten.

14. Vorrichtung nach Anspruch 13, bei welcher der Sensor einen Drucksensor zum unmittelbaren oder mittelbaren Erkennen des gegen die Konstriktionsvorrichtung (2) wirkenden Drucks aufweist.

15. Vorrichtung nach Anspruch 14, ferner mit einer Steuervorrichtung zum Steuern der Betätigungsvorrichtung als Antwort auf Signale des Sensors.

16. Vorrichtung nach Anspruch 13, bei der die Steuervorrichtung eine interne Steuereinheit aufweist, die in den Patienten implantierbar ist, um die Betätigungsvorrichtung als Antwort auf Signale des Sensors zu steuern.

17. Vorrichtung nach einem der Ansprüche 1-14, ferner mit einer Steuervorrichtung zum Steuern der Betätigungsvorrichtung.

18. Vorrichtung nach Anspruch 17, bei der die Steuervorrichtung eine interne Steuereinheit aufweist, die in den Patienten implantierbar ist, um die Betätigungsvorrichtung zu steuern.

19. Vorrichtung nach Anspruch 18, bei der die interne Steuereinheit programmierbar ist.

20. Vorrichtung nach Anspruch 19, bei der die Steuervorrichtung eine externe Steuereinheit außerhalb des Patientenkörpers aufweist, wobei die innere Steuereinheit durch die externe Steuereinheit programmierbar ist.

21. Vorrichtung nach einem der Ansprüche 1-20, ferner mit einem in den Patienten implantierbaren Schalter zum unmittelbaren oder mittelbaren Schalten der von der elektrischen Energiequelle gelieferten elektrischen Energie.

## Revendications

1. Appareil de traitement de l'impuissance sexuelle masculine, comprenant un dispositif de rétrécissement (2) implantable dans un patient homme, qui souffre d'impuissance sexuelle, afin qu'il coopère avec le tissu pénien du patient ou le prolongement de ce tissu, un dispositif électrique implantable de manoeuvre (5) qui manoeuvre le dispositif de rétrécissement pour rétrécir temporairement le tissu pénien ou son prolongement et limiter le débit sanguin sortant du pénis pour provoquer une érection, une alimentation électrique (14) destinée à être implantée dans le patient à distance du dispositif de manoeuvre, et un fil électrique isolé (18) connectant l'alimentation électrique au dispositif de manoeuvre, **caractérisé en ce que** le fil électrique (18) est élastique et s'étend en hélice entre l'alimentation électrique (14) et le dispositif électrique de manoeuvre (5).

2. Appareil selon la revendication 1, comprenant en outre un dispositif implantable (17) de transformation d'énergie capable de transformer l'énergie sans fil transmise depuis l'extérieur du corps du patient en énergie électrique.

3. Appareil selon la revendication 1 ou 2, dans lequel le dispositif de manoeuvre (5) comporte un moteur électrique (7) connecté à l'alimentation électrique (14) par le fil électrique en hélice (18).

4. Appareil selon la revendication 3, dans lequel le dispositif de rétrécissement (2) n'est pas gonflable.

5. Appareil selon la revendication 4, dans lequel le dispositif de rétrécissement (2) comprend un organe allongé (3) de rétrécissement destiné à s'étendre autour du tissu pénien ou de son prolongement.

6. Appareil selon la revendication 5, dans lequel l'organe de rétrécissement (3) comporte une partie principale et deux parties allongées d'extrémité (9, 10), et le dispositif de manoeuvre (5) établit un déplacement relatif longitudinal entre les parties d'extrémité de l'organe de rétrécissement.

7. Appareil selon la revendication 6, dans lequel le dispositif de manoeuvre (5) comprend un moteur électrique et un dispositif (8) de transfert de mouvement en coopération avec l'une au moins (10) des parties d'extrémité de l'organe de rétrécissement (3) et destiné à être manoeuvré par le moteur électrique (7) pour déplacer cette partie d'extrémité (10) par rapport à l'autre partie d'extrémité (9) de l'organe de rétrécissement.

8. Appareil selon la revendication 7, dans lequel le dispositif (8) de transfert de mouvement comprend une roue dentée (11) fixée par rapport à l'autre partie d'extrémité (9) de l'organe de rétrécissement (3), et une crémaillère (13) formée sur la première partie d'extrémité (10) de l'organe de rétrécissement, la roue dentée et la crémaillère étant en prise mutuellement.

9. Appareil selon la revendication 8, dans lequel le dispositif de manoeuvre (5) comprend une vis d'engrenage (12) raccordée entre le moteur électrique (7) et la roue dentée (11).

10. Appareil selon l'une quelconque des revendications 1 à 3, dans lequel le dispositif de rétrécissement (2) comprend un dispositif hydraulique de rétrécissement et le dispositif de manoeuvre comprend une pompe électrique raccordée hydrauliquement au dispositif hydraulique de rétrécissement.

11. Appareil selon l'une quelconque des revendications 5 à 9, dans lequel le dispositif de rétrécissement (2) comprend un boîtier (6) contenant le dispositif de manoeuvre (5), et l'organe allongé de rétrécissement (3) et le boîtier sont destinés à former une boucle autour du tissu pénien du patient ou de son prolongement.

12. Appareil selon l'une quelconque des revendications 1 à 11, comprenant en outre une commande à distance sans fil (16) destinée à commander le dispositif de manoeuvre (5) pour la manoeuvre du dispositif de rétrécissement (2).

13. Appareil selon l'une quelconque des revendications 1 à 12, comprenant en outre au moins un capteur implantable destiné à détecter au moins un paramètre physique du patient.

14. Appareil selon la revendication 13, dans lequel le capteur comprend un capteur de pression destiné à détecter directement ou indirectement la pression exercée contre le dispositif de rétrécissement (2).

15. Appareil selon la revendication 14, comprenant en outre un dispositif de commande du dispositif de manoeuvre en fonction de signaux provenant du capteur.

16. Appareil selon la revendication 15, dans lequel le dispositif de commande comprend une unité interne de commande implantable dans le patient pour la commande du dispositif de manoeuvre en fonction de signaux provenant du capteur.

17. Appareil selon l'une quelconque des revendications 1 à 14, comprenant en outre un dispositif de commande de dispositif de manoeuvre.

18. Appareil selon la revendication 17, dans lequel le dispositif de commande comprend une unité interne de commande implantable dans le patient pour la commande du dispositif de manoeuvre.

19. Appareil selon la revendication 18, dans lequel l'unité interne de commande est programmable.

20. Appareil selon la revendication 19, dans lequel le dispositif de commande comprend une unité externe de commande placée à l'extérieur du corps du patient, l'unité interne de commande étant programmable par l'unité externe de commande.

21. Appareil selon l'une quelconque des revendications 1 à 20, comprenant en outre un interrupteur implantable dans le patient et destiné à commuter directement ou indirectement l'énergie électrique transmise par l'alimentation électrique.
